# EUROPEAN PATENT SPECIFICATION

(11) **EP 2 490 601 B1**
(45) Date of publication and mention of the grant of the patent: **08.07.2015**
(21) Application number: 10768348.4
(22) Date of filing: 13.10.2010
(51) Int. Cl.: A61B 17/12, A61B 17/00, A61B 19/00, A61M 25/10

(54) **BALLOON-TIPPED ENDOSCOPIC SYSTEM WITH INVERTED SLEEVE**
ENDOSKOPSYSTEM MIT BALLONSPITZE UND UMGEKEHRTER HÜLSE
SYSTÈME ENDOSCOPIQUE À BALLONNET MONTÉ EN EXTRÉMITÉ ET À MANCHON INVERSÉ

(30) Priority: 19.10.2009 US 252981 P
(43) Date of publication of application: 29.08.2012
(73) Proprietor: Cook Medical Technologies LLC, Bloomington, IN 47404 (US)
(72) Inventor: DUCHARME, Richard, W., Winston-salem, NC 27106 (US); MCLAWHORN, Tyler, E., Winston-salem, NC 27105 (US); SURTI, Vihar, C., Winston-salem, NC 27104 (US)
(74) Representative: Garratt, Peter Douglas
(86) International application number: PCT/US2010/052444
(87) International publication number: WO 2011/049795

(56) References cited:
- US-A- 6 059 809
- US-A1- 2005 131 446
- US-A1- 2006 004 439
- US-A1- 2006 009 798

## Description

### TECHNICAL FIELD

The present invention relates to ballooned-tipped endoscopic devices useful in natural orifice transluminal endoscopy surgery. The systems can be used to deploy therapeutic devices and obtain tissue samples.

### BACKGROUND

Openings or perforations in the walls of internal organs and vessels may be naturally occurring, or formed intentionally or unintentionally. These openings may be used to gain access to adjacent structures of the body, such techniques being commonly referred to as transluminal procedures. For example, culdoscopy was developed over 70 years ago, and involves transvaginally accessing the peritoneal cavity by forming an opening in the cul de sac. This access to the peritoneal cavity allows medical professionals to visually inspect numerous anatomical structures, as well as perform various procedures such as biopsies or other operations, such as tubal ligation. Many transluminal procedures for gaining access to various body cavities using other bodily lumens have also been developed. One field of procedures has been referred to as Natural Orifice Transluminal Endoscopy Surgery ("NOTES"). Natural orifices such as the mouth, nose, ear, anus, or vagina may provide access to such bodily lumens and cavities. The bodily lumen(s) of the gastrointestinal tract are often endoscopically explored and can be utilized to provide access to the peritoneal cavity and other body cavities, all in a minimally invasive manner. U.S. Patent Publication No. 2008/0132948 discloses such a procedure. Reference is also directed to US 2006/004439 which discloses an arrangement of an outer catheter, a movable inner catheter and a balloon-tipped catheter within the inner catheter for deploying a device. The latter document discloses the preamble of claim 1.

Compared to traditional open surgery or laparoscopic surgery, transluminal procedures are less invasive by eliminating abdominal incisions (or other exterior incisions) and incision related complications, while also reducing postoperative recovery time, reducing pain, and improving cosmetic appearance. At the same time, there remain challenges to transluminal procedures, including providing a suitable conduit to the openings and body cavities, robust medical devices that are maneuverable via the conduit and operable within the body cavity, sterility of the conduit, maintaining insufflation of the body cavity, proper closure of the opening and prevention of infection. For example, when an opening is formed in a bodily wall of the gastrointestinal tract, such as in the stomach or intestines, spillage of the stomach contents, intestinal contents or other bodily fluids into the adjacent body cavity can occur. Travel of bacteria laden fluids outside of the gastrointestinal tract may cause unwanted and sometimes deadly infection.

One of the current challenges in NOTES procedures is sterile delivery of a material into the peritoneum and obtaining tissue samples in a sterile way.

### BRIEF SUMMARY

The invention is defined in claim 1. Herein provided is a multi-luminal system comprising an outer catheter comprising a distal portion and a wall that encloses an outer lumen; an inner catheter movably disposed within the outer lumen and having an inner lumen; a balloon-tipped catheter movably disposed within the inner lumen and having a distal portion and a proximal portion, wherein the distal portion of the balloon-tipped catheter comprises a balloon; a deployable device within the outer lumen. The system can also comprise an invertible sleeve within the outer lumen with a first section attached to the distal portion of the outer catheter and a second section attached to a push mechanism that is proximal to the deployable device. The balloon is expandable to contact the invertible sleeve to provide a seal to prevent fluids from entering the outer lumen.

The system can also comprise a push mechanism that is a push catheter having a lumen therethrough located within the outer lumen with a first position proximal to the balloon. The deployable device can be a medical device that provides a therapeutic treatment to an animal body. The deployable device is about the inner catheter and the inner catheter further comprises a push mechanism for deploying the deployable device.

Described herein is also a delivery system wherein the proximal portion of the balloon-tipped catheter is an elongated catheter shaft within the inner lumen and the balloon has a first predetermined diameter when inflated and a second predetermined diameter when deflated. The catheters can be concentric with the inner lumen of the inner catheter located within the lumen of the push catheter. The inner catheter and push catheter may not be concentric. The invertible sleeve can be comprised of biocompatible cloth or fabric mesh.

Also described herein is a method of delivery using a multi-luminal delivery system described herein. The method comprises introducing the system into an endoluminal vessel until the balloon reaches a desired location; deflating the balloon; placing the deflated balloon within the inner lumen of the inner catheter; manipulating the invertible sleeve such that the second section is distal to the first section; and deploying the deployable device by manipulating the outer catheter relative to the inner catheter such that the deployable device is distal to the outer catheter.

### BRIEF DESCRIPTION OF THE DRAWINGS

Figure 1 is a cutaway perspective view of the delivery system with an inflated balloon and invertible sleeve.
Figure 2 is a cutaway perspective view of the delivery system with a deflated balloon.
Figure 3 is a perspective view of a hernia mesh being advanced by the push catheter and the invertible sleeve being deployed.
Figure 4 is a cutaway perspective view of the delivery system with the hernia mesh deployed and the push catheter retracted into the system.

### DETAILED DESCRIPTION OF THE DRAWINGS AND THE PRESENTLY PREFERRED EMBODIMENTS

The term "prosthesis" means any replacement for a body part or for a function of that body part or any device that enhances or adds functionality to a physiological system.

The term "stent" means any device that provides rigidity, expansion force, or support to a prosthesis, such as a stent graft. In one configuration, the stent may represent a plurality of discontinuous devices. In another configuration, the stent may represent one device. Stents may have a wide variety of configurations and may be balloon-expandable or self-expanding. Typically, stents have a circular cross-section when fully expanded, so as to conform to the generally circular cross-section of a body lumen. In one example, a stent may comprise struts (elongate portions) and acute bends (curvilinear portions) that are arranged in a zigzag configuration in which the struts are set at angles to each other and are connected by the acute bends. Although an undulating configuration is used throughout this application, it is understood that the stent may have a sinusoidal or a zigzag configuration as well. One example of a stent configuration is a Z-stent. The stents as described in this disclosure may be attached to the exterior of the graft, the interior of the graft, and/or may be sandwiched between two or more layers of graft material.

A variety of biocompatible materials may be employed to construct the stent, or portions of the stent, including metals and/or alloys, medically acceptable polymers, and/or bioabsorbable polymers or materials. The metals and/or alloys may, among other things, include stainless steel, tantalum, nitinol, gold, silver, tungsten, platinum, inconel, cobalt-chromium alloys, and iridium, all of which are commercially available metals or alloys used in the fabrication of medical devices. In a preferred configuration, the stent is constructed from nitinol, stainless steel, and/or cobalt-chromium alloys.

The term "graft or graft material" means a generally cannular or tubular member which acts as an artificial vessel or prosthesis. A graft by itself or with the addition of other elements, such as structural components, can be an endoluminal prosthesis. The graft comprises a single material, a blend of materials, a weave, a laminate, or a composite of two or more materials.

The term "catheter" generally means medical devices including balloon-tipped catheters, guide catheters, and delivery catheters.

The term "deployable device" generally means a medical device that provides therapeutic treatment to a medically treatable area of an animal body. Deployable devices include, but are not limited to, a hernia mesh, ligating barrel, jejunal magnet, or stent graft.

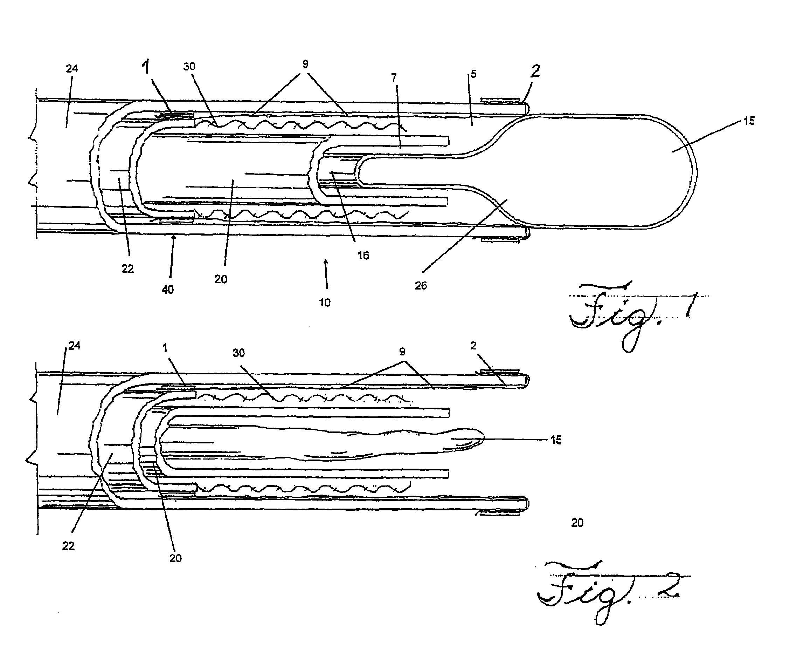

Figure 1 shows an embodiment of multi-luminal delivery system 10 that minimizes the introduction of bacteria into the peritoneum and the risk of contaminating a sterile deployable device. The system is comprised of catheters that are roughly concentric and contain a deployable device that may be too large for placement in the access channel of an endoscope. The system can be introduced into the gastrointestinal tract through the mouth or other natural bodily orifice. Figure 1 illustrates the delivery system **10** in the initial configuration suitable for introduction into the patient and advancement to the target site (i.e., where the deployable device is to be deployed).

As shown in Figure 1, the system **10** comprises: an outer catheter **24,** an inner catheter **20,** a push catheter **22,** a balloon-tipped catheter **26,** and an invertible sleeve **9**. The outer catheter **24** comprises a wall that encloses an outer lumen **5**. The inner catheter **20** is movably disposed within the outer lumen **5** and has an inner lumen **7** that contains, at least partially, the balloon-tipped catheter **26**. The distal portion of the inner catheter **20** can flare outwardly (not shown) to prevent the accumulation of potentially harmful bacteria from collecting on the outside of the inner catheter **20** and from contacting the sterile deployable device **30**.

The invertible sleeve has a first section **2** that is attached to the distal portion of the outer catheter and a second section **1** that is attached to the push catheter **22**. As shown in Figure 1, the first section **2** of the invertible sleeve **9** is distal to the hernia mesh when the delivery device **10** is in the initial configuration. The distal portion of the balloon-tipped catheter **26** has a balloon **15** that contacts the invertible sleeve **9** near the first section **2**. The seal provided by the contact of the balloon **15** and the invertible sleeve **9** helps to prevent bacteria or other potentially harmful elements from entering the outer lumen **5** or contaminating the deployable device **30**. Although the embodiment illustrated in Figure 1 comprises a balloon-tipped catheter **26** having an expandable balloon **15**, the expandable member could be provided on the inner catheter **20** or on the invertible sleeve **9**. So long as the expandable member is configured to form a seal sufficient to prevent contamination of the outer lumen **5**.

Balloon-tipped catheters are manufactured in a variety of arrangements. The ballooned-tipped catheter **26** has a proximal elongated catheter shaft **16** that is movably disposed within the inner lumen **7** of the inner catheter **20**. It is understood that the distal portion of the balloon-tipped catheter **26**, the actual balloon **15**, has a first larger diameter when inflated (Figure 1) and a second smaller diameter when deflated (Figure 2). As shown in Figure 1, the inflated balloon **15** can contact the invertible sleeve **9** at the distal end of the outer catheter **24** such that blood and other bodily fluids are prevented from entering the outer lumen **5** and contacting the contents of the outer catheter as well as the deployable device **30**. Figure 2 shows the system with the balloon **15** deflated and retracted within the inner catheter **20.**

The balloon-tipped catheter **26** can be made of materials capable of elastic expansion typically used in the field. For example, the balloon-tipped catheter **26** can comprise silicone, latex, or any other suitable material commonly used in the field. The balloon **15** can be tapered, bulbous, or cylindrical. The balloon-tipped catheter **26** can comprise a wire guide to assist in guiding the entire system **10** throughout the gastrointestinal tract. The balloon **15** can have a nipple like tip (not shown) to assist in advancing the catheter. Suitable alternative configurations for the balloon-tipped catheter **26** are disclosed in U.S. Provisional Application No. 61/141,568.

As explained above, the invertible sleeve **9** assists in preventing contamination of the deployable device and the outer lumen. Without the invertible sleeve **9**, the junction of the balloon **15** and the internal wall of the distal portion of the outer catheter **24** may come into contact with bodily fluids and bacteria as the system travels to a desired location. It may be possible that the deployable device may come into contact with remnants of these bodily fluids as it moves past the lip of the outer catheter **24**. As shown in Figure 1, the invertible sleeve **9** contacts the balloon **15** and is between the balloon **15** and the internal wall of the catheter **24**. The first or distal section **2** of the invertible sleeve **9** is attached to the distal portion of the outer catheter **24** while the second or proximal section **1** of the invertible sleeve **9** is attached to the push catheter **22** at a point proximal to the deployable device **30**. Alternatively, the proximal section **1** may be attached to the inner catheter **20**. For example, the proximal section **1** of the invertible sleeve **9** may be attached to a push mechanism disposed on or about the inner catheter **20** proximal to the deployable device **30**. The proximal section **1** may also be left unattached.

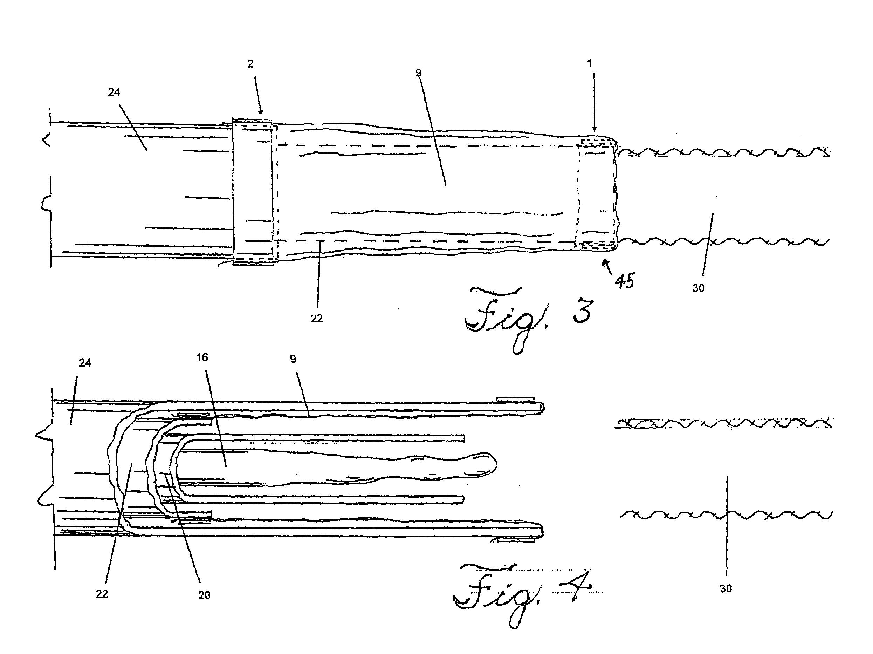

With any of the above configurations, any bacteria will come into contact with the inwardly facing side of the invertible sleeve **9**. When the deployable device **30** is deployed as shown in Figures 3 and 4, the invertible sleeve **9** inverts its position such that the distal section **2** of the invertible sleeve **9** becomes proximal to the proximal section **1** of the invertible sleeve **9** and the side of the invertible sleeve **9** that was once inwardly facing becomes outwardly facing. The push catheter is shown in Figure 3 with dashed lines **22**. As a result, the deployable device **30** can be spared bacterial contact while being implanted.

The push catheter **22** is within the outer lumen and generally has the same diameter as the deployable device **30**. The deployable device shown in the figures is a hernia mesh **30**. The push catheter **22** is shown in a first position in Figure 1. This first position **40** is proximal to the balloon **15**. The first position **40** is generally the initial position of the push catheter **22** when the system **10** is inserted into the human body. The push catheter **22** is in the first position **40** when the deployable device **30** is not yet delivered or advanced/deployed out of the outer catheter **24** and the balloon **15** is still expanded and in contact with the wall of the outer catheter **24**. Figure 2 shows the balloon **15** deflated and withdrawn into the inner catheter **20**. The push catheter's **22** second position **45** is obtained when it is advanced to deliver the deployable device **30**, as shown in Figure 3. Here, the second position **45** is distal to the balloon **15** and the outer catheter **24** such that the hernia mesh **30** is completely clear of the outer catheter **30**. The distal end of the inner catheter **20** can be aligned with the distal end of the outer catheter 24 so as to not obstruct the delivery of the delivery device **30** as it is pushed by the push catheter **22** in a distal direction. Alternatively, the deployable device **30** may be delivered by retracting the outer catheter **24** while holding the pusher catheter **22** and the inner catheter **20** stationary.

The proximal elongated shaft **16** of the ballooned-tipped catheter **26** is disposed within the inner lumen **7** of the inner catheter **20**. Once the delivery device **10** is advanced to the target site with the patient and the balloon **15** is deflated, it is retracted into the inner lumen **7**. While within the inner catheter **20**, the balloon **15** and any other bodily fluids disposed thereon are maintained separate from the deployable device **30**. Contact between the deployable device **30** and the balloon **15** is minimized with the aid of the invertible sleeve **9** so as to maintain the sterility of the deployable device **30** until implantation. The seal provided by the balloon **15** minimizes the possibility of transferring bacteria or other microorganisms that may be considered harmful from a first environment into a second environment. The system **10** travels through the colon and out of an incision into the peritoneum. The system **10** may be exposed to fluids and bacteria in both environments. The seal helps prevent material found in the colon from entering the outer lumen **5** and being transferred to a second environment, such as the peritoneum. Once the balloon **15** is withdrawn inside the inner catheter **20**, the push catheter **22** can be advanced distally to deploy the deployable device, the hernia mesh **30**, out of the outer catheter **24**. Alternatively, the outer catheter 24 can be retracted to deploy the deployable device **30**.

The deployable device **30** and the push catheter **22** have smaller diameters than the outer catheter **24** but larger diameters than the inner catheter **20**. Although a hernia mesh **30** is shown in these figures as the deployable device, other devices can be delivered using this system. For example, the delivery system **10** can also be used to deliver, for example, gauzes of any type, large volumes of fluid or powders, specimen retrieval bags, or slings. The deployable device can be a stent graft, ligating bands, or jejunal magnets. The deployable device can be any device used in endoscopy but is too large to fit in the accessory channel of an endoscope. The deployable devices may require other accoutrements for delivery. For instance, if actuating wires are needed for delivery of a stent graft they can be carried within the push catheter **22**. Similarly, activation lines may be carried within the push catheter **22** for delivering ligating bands.

The system **10** can also comprise a wire guide to assist in delivery, such as is disclosed in U.S. Patent Publication No. 2010/0168612. The system can also be adapted to accommodate joystick manipulation.

In an alternative embodiment, the push catheter **22** and the inner catheter **20** comprise a unitary construction. Or the push catheter **22** may be eliminated from the delivery system 10 altogether. With respect to the latter configuration, the function of the push catheter **22** may be replaced by a push mechanism disposed on the inner catheter **20** as described below.

This inner catheter **20** may comprise a push mechanism to assist in deploying the deployable device **30**. The push mechanism can be a ridge having a diameter slightly larger than the diameter of the inner catheter **20** that proximally abuts the proximal end of a deployable device **30**. The ridge can be used to advance the deployable device **30** out of the outer lumen **5** or hold the deployable device **30** in place while the outer catheter **24** is retracted. The ridge can be radiopaque. The balloon tip **15** can also comprise a wire guide to assist in placing the system **10**.

The system is used to deliver deployable devices through a natural bodily orifice, such as the mouth, nose, or anus, with the balloon inflated to seal off bodily fluids. Through the mouth, the system would be able to access the upper gastrointestinal tract, the stomach, the duodenum, and the small intestine. Through the anus, the system could access the colon, which includes the large and small intestine. A system having comparable diameter can access the sinuses through the nose. Other areas of the body can be accessed with internal incisions in the stomach, vagina, bladder, or colon to perform procedures such as appendectomies, gastric revisions, ligation, or biopsies.

The system **10** is introduced into a human anus and advanced through the rectum and into the colon until the balloon tip **15** reaches a desired location. The system can be introduced transrectally, transvaginally, or transgastrically. The balloon tip **15** provides a seal to prevent bodily fluids from entering the outer lumen and contaminating the sterile deployable device. Because of its flexibility and contour, the balloon-tipped catheter **26** acts as a flexible tip to the system **10** that prevents damage to the anatomy as the system **10** winds its way through the gastrointestinal tract to the desired location.

Additional details of the components of the delivery system **10**, and exemplary methods of using the system to deliver deployable devices, are disclosed in U.S. Patent Publication No. 2010/0168612.

The foregoing description of various embodiments of the invention has been presented for purposes of illustration and description. It is not intended to be exhaustive or to limit the invention to the precise embodiments disclosed. Numerous modifications or variations are possible in light of the above teachings. The embodiments discussed were chosen and described to provide the best illustration of the principles of the invention and its practical application to thereby enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally, and equitably entitled.

## Claims

1. A multi-luminal delivery system comprising:
an outer catheter (24) comprising a distal portion and a wall that encloses an outer lumen (5);
an inner catheter (22) movably disposed within the outer lumen and having an inner lumen (7);
a balloon-tipped catheter (26) movably disposed within the inner lumen and having a distal portion and a proximal portion, wherein the distal portion of the balloon- tipped catheter comprises a balloon; and
a deployable device (30) within the outer lumen;
**characterised by** an invertible sleeve (9) removably disposed within the outer lumen and having a first section (2) attached to the distal portion of the outer catheter and a second section (1) disposed proximal to the deployable device, the sleeve being disposed about the deployable device,
wherein the balloon is expandable to contact the invertible sleeve to provide a seal to prevent bodily fluids from entering the outer lumen.

2. The delivery system of claim 1 further comprising a push mechanism disposed proximally of the deployable device and configured to engage the deployable device during deployment.

3. The delivery system of claim 2 wherein the push mechanism comprises a push catheter movably disposed within the outer lumen and positioned with a first position proximal to the balloon.

4. The delivery system of claim 3 wherein the inner catheter and the outer catheter are concentric, and wherein the inner catheter is disposed within a lumen of the push catheter.

5. The delivery system of claim 3 wherein the inner catheter and push catheter are not concentric.

6. The delivery system of claim 3 wherein the second section of the sleeve is attached to the push catheter.

7. The delivery system of claim 2 wherein the push mechanism comprises a ridge disposed about the inner catheter and positioned proximal to the balloon.

8. The delivery system of claim 5 wherein the second section of the sleeve is attached to the inner catheter.

9. The delivery system of claim 1 wherein the deployable device is a medical device that provides a therapeutic treatment to an animal body.

10. The delivery system of claim 1 wherein the deployable device is disposed about the inner catheter, and wherein the inner catheter further comprises a push mechanism for engaging the deployable device.

11. The delivery system of claim 1 wherein the proximal portion of the balloon-tipped catheter is an elongated catheter shaft movably disposed within the inner lumen, and wherein the balloon has a first predetermined diameter when inflated and a second predetermined diameter when deflated, the first predetermined diameter being sufficient to contact and form a seal with the sleeve.

12. The delivery system of claim 1 wherein the invertible sleeve is comprised of biocompatible cloth or fabric mesh.

13. The delivery system of claim 1 wherein the push mechanism comprises radiopaque markers.

## Patentansprüche

1. Mehrlumiges Verabreichungssystem, umfassend:
einen Außenkatheter (24) mit einem distalen Abschnitt und einer Wand, die ein Außenlumen (5) umschließt;
einen Innenkatheter (22), der beweglich in dem Außenlumen angeordnet ist und ein Innenlumen (7) aufweist;
einen Katheter (26) mit Ballonspitze, der in dem Innenlumen angeordnet ist und einen distalen Abschnitt und einen proximalen Abschnitt aufweist, worin der distale Abschnitt des Katheters mit Ballonspitze einen Ballon umfasst; und
eine einsetzbare Vorrichtung (30) in dem Außenlumen;
**gekennzeichnet durch** eine umkehrbare Hülse (9), die entfernbar in dem Außenlumen angeordnet ist und einen ersten, an dem distalen Abschnitt des Außenkatheters befestigten Abschnitt (2) und einen zweiten, proximal zu der einsetzbaren Vorrichtung angeordneten Abschnitt (1) aufweist, wobei die Hülse um die einsetzbare Vorrichtung angeordnet ist,
worin der Ballon ausdehnbar ist, um die umkehrbare Hülse zu berühren und zur Verhinderung des Eindringens von Körperflüssigkeiten in das Außenlumen eine Dichtung bereitzustellen.

2. Verabreichungssystem nach Anspruch 1, ferner einen Schiebemechanismus umfassend, der proximal zu der einsetzbaren Vorrichtung angeordnet und für den Eingriff mit der einsetzbaren Vorrichtung während des Einsatzes ausgelegt ist.

3. Verabreichungssystem nach Anspruch 2, worin der Schiebemechanismus einen Schiebekatheter umfasst, der beweglich in dem Außenlumen angeordnet ist und in einer ersten Stellung proximal zu dem Ballon platziert ist.

4. Verabreichungssystem nach Anspruch 3, worin der Innenkatheter und der Außenkatheter konzentrisch sind und worin der Innenkatheter in einem Lumen des Schiebekatheters angeordnet ist.

5. Verabreichungssystem nach Anspruch 3, worin der Innenkatheter und der Schiebekatheter nicht konzentrisch sind.

6. Verabreichungssystem nach Anspruch 3, worin der zweite Abschnitt der Hülse an dem Schiebekatheter befestigt ist.

7. Verabreichungssystem nach Anspruch 2, worin der Schiebemechanismus eine Rippe umfasst, die um den Innenkatheter angeordnet und proximal zu dem Ballon platziert ist.

8. Verabreichungssystem nach Anspruch 5, worin der zweite Abschnitt der Hülse an dem Innenkatheter befestigt ist.

9. Verabreichungssystem nach Anspruch 1, worin die einsetzbare Vorrichtung eine medizinische Vorrichtung ist, die eine therapeutische Behandlung für einen tierischen Körper bereitstellt.

10. Verabreichungssystem nach Anspruch 1, worin die einsetzbare Vorrichtung um den Innenkatheter angeordnet ist und worin der Innenkatheter ferner einen Schiebemechanismus für den Eingriff mit der einsetzbaren Vorrichtung umfasst.

11. Verabreichungssystem nach Anspruch 1, worin der proximale Abschnitt des Katheters mit Ballonspitze ein langgestreckter Katheterschaft ist, der beweglich in dem Innenlumen angeordnet ist, und worin der Ballon einen ersten vorbestimmten Durchmesser nach dem Aufblasen und einen zweiten vorbestimmten Durchmesser nach dem Entleeren aufweist, wobei der erste vorbestimmte Durchmesser für Kontakt und Bildung einer Dichtung mit der Hülse ausreicht.

12. Verabreichungssystem nach Anspruch 1, worin die umkehrbare Hülse aus einem biokompatiblen Tuch oder Gittergewebe besteht.

13. Verabreichungssystem nach Anspruch 1, worin der Schiebemechanismus röntgenopake Markierungen umfasst.

## Revendications

1. Système de distribution à plusieurs lumières, comprenant :
un cathéter extérieur (24) comprenant une portion distale et une paroi entourant une lumière extérieure (5) ;
un cathéter intérieur (22) disposé de manière mobile à l'intérieur de la lumière extérieure et présentant une lumière intérieure (7) ;
un cathéter à pointe à ballonnet (26) disposé de manière mobile à l'intérieur de la lumière intérieure et ayant une portion distale et une portion proximale, la portion distale du cathéter à pointe à ballonnet comprenant un ballonnet ; et
un dispositif déployable (30) à l'intérieur de la lumière extérieure ;
**caractérisé par** un manchon pouvant être inversé (9) disposé de manière amovible à l'intérieur de la lumière extérieure et présentant une première section (2) attachée à la portion distale du cathéter extérieur et une deuxième section (1) disposée à proximité du dispositif déployable, le manchon étant disposé autour dudit dispositif déployable,
le ballonnet pouvant être gonflé de manière à venir en contact avec le manchon pouvant être inversé afin d'assurer un joint pour empêcher que des fluides corporels ne pénètrent à l'intérieur de la lumière extérieure.

2. Système de distribution selon la revendication 1, comprenant en outre un mécanisme de poussée disposé à proximité du dispositif déployable et configuré pour venir en prise avec le dispositif déployable au cours du déploiement.

3. Système de distribution selon la revendication 2, dans lequel le mécanisme de poussée comprend un cathéter de poussée disposé de manière mobile à l'intérieur de la lumière extérieure et positionné avec une première position à proximité du ballonnet.

4. Système de distribution selon la revendication 3, dans lequel le cathéter intérieur et le cathéter extérieur sont concentriques, et dans lequel le cathéter intérieur est disposé à l'intérieur d'une lumière du cathéter de poussée.

5. Système de distribution selon la revendication 3, dans lequel le cathéter intérieur et le cathéter de poussée ne sont pas concentriques.

6. Système de distribution selon la revendication 3, dans lequel la deuxième section du manchon est attachée au cathéter de poussée.

7. Système de distribution selon la revendication 2, dans lequel le mécanisme de poussée comprend une arête disposée autour du cathéter intérieur et positionnée à proximité du ballonnet.

8. Système de distribution selon la revendication 5, dans lequel la deuxième section du manchon est attachée au cathéter intérieur.

9. Système de distribution selon la revendication 1, dans lequel le dispositif déployable est un dispositif médical qui fournit un traitement thérapeutique à un corps animal.

10. Système de distribution selon la revendication 1, dans lequel le dispositif déployable est disposé autour du cathéter intérieur, et dans lequel le cathéter intérieur comprend en outre un mécanisme de poussée pour venir en prise avec le dispositif déployable.

11. Système de distribution selon la revendication 1, dans lequel la portion proximale du cathéter à pointe à ballonnet est une tige de cathéter allongée disposée de manière mobile à l'intérieur de la lumière intérieure, et dans lequel le ballonnet présente un premier diamètre prédéterminé lorsqu'il est gonflé et un deuxième diamètre prédéterminé lorsqu'il est dégonflé, le premier diamètre prédéterminé étant suffisant pour venir en contact avec le manchon et former un joint avec celui-ci.

12. Système de distribution selon la revendication 1, dans lequel le manchon pouvant être inversé est constitué d'une toile biocompatible ou d'un maillage en tissu.

13. Système de distribution selon la revendication 1, dans lequel le mécanisme de poussée comprend des marqueurs radio-opaques.
